(19) Europäisches Patentamt
European Patent Office
Office européen des brevets

(11) **EP 3 928 971 B1**

(12) **EUROPÄISCHE PATENTSCHRIFT**

(45) Veröffentlichungstag und Bekanntmachung des Hinweises auf die Patenterteilung:
**04.10.2023  Patentblatt 2023/40**

(21) Anmeldenummer: **21174945.2**

(22) Anmeldetag: **20.05.2021**

(51) Internationale Patentklassifikation (IPC):
**B30B 15/26** *(2006.01)*      **G07C 3/14** *(2006.01)*

(52) Gemeinsame Patentklassifikation (CPC):
**B30B 15/26; G07C 3/14;** B30B 11/08

(54) **VERFAHREN ZUR PRODUKTVERFOLGUNG IN EINER ANLAGE**

METHOD FOR TRACKING PRODUCTS IN A SYSTEM

PROCÉDÉ DE SUIVI DES PRODUITS DANS UNE INSTALLATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.06.2020   DE 102020115777**

(43) Veröffentlichungstag der Anmeldung:
**29.12.2021   Patentblatt 2021/52**

(73) Patentinhaber: **Fette Compacting GmbH
21493 Schwarzenbek (DE)**

(72) Erfinder:
• **Walter, Nicolas
22143 Hamburg (DE)**
• **Evers, Alexander
22941 Bargteheide (DE)**

(74) Vertreter: **Hauck Patentanwaltspartnerschaft mbB
Postfach 11 31 53
20431 Hamburg (DE)**

(56) Entgegenhaltungen:
**EP-A1- 2 427 166        EP-B1- 2 427 166
WO-A1-2008/149190   DE-A1-102016 218 135
US-A- 5 114 630         US-A1- 2010 161 102**

**Beschreibung**

Die Erfindung betrifft ein Verfahren zur Produktverfolgung in einer Anlage zur kontinuierlichen Verarbeitung pulverförmigen Produkts zu Erzeugnissen.

In solchen Anlagen werden pulverförmige Produkte zu Tabletten oder Kapseln verarbeitet. Entsprechend umfassen die Anlagen eine Tablettenpresse oder eine Kapselfüllmaschine. Solche Anlagen weisen einen oder mehrere Produkteinlässe für in der Anlage zu verarbeitendes pulverförmiges Produkt auf. Sofern mehrere Produkteinlässe für unterschiedliche Produkte vorgesehen sind, weisen derartige Anlagen üblicherweise weiterhin eine Mischeinrichtung auf, in der die Produkte zu dem zu verpressenden Produkt vermischt werden. Aus der Mischeinrichtung wird das gemischte Produkt dann zum Beispiel einer Tablettenpresse, beispielsweise einer Rundläufertablettenpresse, zugeführt, wo die Produktmischung zu Tabletten verpresst wird. Über einen

Tablettenauslass der Tablettenpresse gelangen die fertig hergestellten Tabletten aus der Anlage. Solche Anlagen können als kontinuierliche Anlagen ausgebildet sein, bei denen das der Anlage kontinuierlich zugeführte Produkt im Gegensatz zu einem Batch-Prozess kontinuierlich verarbeitet wird, zum Beispiel zu Tabletten oder Kapseln. Weiterhin sind sogenannte Containment-Anlagen bekannt, die durch besondere Abdichtungsmaßnahmen einen Austritt von Produktstaub in die Umgebung weitgehend verhindern.

Insbesondere bei kontinuierlich arbeitenden Anlagen besteht ein Bedürfnis, das der Anlage am Einlass zugeführte Produkt in der Anlage hergestellten Erzeugnissen zuzuordnen. Dies ist erforderlich, wenn das Produkt zum Beispiel durch Sensoren der Anlage als fehlerhaft erkannt wird. In diesem Fall müssen die aus diesem Produkt hergestellten Erzeugnisse, zum Beispiel Tabletten oder Kapseln, aussortiert werden, wobei zur Vermeidung unnötigen Ausschusses möglichst nur die aus der entsprechenden Produktcharge hergestellten Erzeugnisse aussortiert werden sollen. Es besteht entsprechend ein Bedürfnis zur Produktverfolgung in der Anlage.

Zur Produktverfolgung sind sogenannte Verweilzeitmodelle bekannt, bei denen anhand der Verweil- bzw. Durchlaufzeit des Produkts durch die Anlage die hergestellten Erzeugnisse dem in die Anlage zugeführten Produkt zugeordnet werden. Beispielsweise aus EP 2 427 166 B1 ist ein Verfahren zur kontinuierlichen Herstellung von Tabletten bekannt, bei dem eine Produktverfolgung in der Anlage mittels Zeitstempeln erfolgen soll. Die Produktverfolgung auf Grundlage solcher Verweilzeitmodelle ist allerdings insbesondere bei Zuführen mehrerer unterschiedlicher Produkte in die Anlage kompliziert. So kann sich die Verweilzeit abhängig von den zugeführten Produkten unterscheiden. Die Verweilzeit muss entsprechend für jede Produktkombination neu ermittelt werden, um zuverlässige Ergebnisse zu erlangen. Außerdem ist die Genauigkeit von Verweilzeitmodellen stark abhängig von dem Betriebspunkt der Anlage, also den jeweils eingestellten Betriebsparametern. Unmittelbar ersichtlich ist dies natürlich für die Produktionsgeschwindigkeit der Anlage. Die Verweilzeit hängt aber auch von weiteren Betriebsparametern ab, beispielsweise der Betriebsart einer Mischeinrichtung. Bereits kleine Abweichungen von einem Betriebspunkt müssen in dem Verweilzeitmodell berücksichtigt werden. Wie eine solche Abweichung von einem Betriebspunkt sich auf die Verweilzeit auswirkt, muss jeweils neu experimentell ermittelt werden, insbesondere auch abhängig von dem jeweiligen Produkt. Der Aufwand zur Parametrierung eines Verweilzeitmodells, um eine akzeptable Genauigkeit zu erreichen, ist daher sehr hoch.

Aus DE 10 2016 218 135 A1 ist ein Produktionsmodul zum Herstellen fester Arzneiformen bekannt, bei dem das in die Anlage gegebene Produkt mittels Auffangschleusen und Sperrventilen in separate Lots aufgeteilt wird, die einzeln und ohne Fluidkommunikation zu anderen Lots die Anlage durchlaufen. Durch die Aufteilung des Produkts in Lots soll eine Rückvermischung von Produkt verhindert und eine Echtzeit-Verfolgung der Lots in der Anlage ermöglicht werden.

Aus WO 2008/149190 A1 sind eine Dosiervorrichtung und ein Verfahren zum Mischen von in einer Mehrzahl von Hoppern befindlichen Materialien bekannt.

Aus US 5 114 630 A ist ein Verfahren zum kontinuierlichen Gießen von Materialien bekannt, bei dem die Zusammensetzungsverhältnisse von Materialmengen gemessen werden und außerhalb der Spezifikation liegende Materialmengen ausgeschleust werden.

Ausgehend von dem erläuterten Stand der Technik liegt der Erfindung die Aufgabe zugrunde, ein Verfahren der eingangs genannten Art bereitzustellen, mit dem in einfacher und präziser Weise eine Produktverfolgung in einer Anlage, in der pulverförmiges Produkt zu Erzeugnissen verarbeitet wird, möglich ist.

Die Erfindung löst die Aufgabe durch den Gegenstand von Anspruch 1. Vorteilhafte Ausgestaltungen finden sich in den abhängigen Ansprüchen, der Beschreibung und den Figuren.

Für ein Verfahren der eingangs genannten Art löst die Erfindung die Aufgabe dadurch, dass das in die Anlage eingegebene Produkt anhand von Messdaten mindestens eines an einem Produkteinlass der Anlage angeordneten Einlassmassesensors in Masseeinheiten aufgeteilt wird, und dass der Fortgang der Masseeinheiten in der Anlage anhand von Messdaten mindestens eines weiteren Massesensors der Anlage verfolgt wird.

Die Anlage umfasst einen oder mehrere Produkteinlässe für in der Anlage zu verarbeitendes Produkt. Die Anlage kann weiterhin eine Mischeinrichtung umfassen zum Vermischen unterschiedlicher der Anlage zugeführter Produkte. Die Anlage umfasst darüber hinaus eine Produktionsmaschine, eine Tablettenpresse, beispielsweise eine Rundläufertablettenpresse, und/ oder eine Kapselfüllmaschine, in der das der Anlage zugeführte Produkt bzw. die in der Mischeinrichtung hergestellte Produktmischung zu Erzeugnissen, also Tabletten oder Kapseln, verarbeitet wird. Die hergestellten Erzeugnisse können zum Beispiel orale feste Darreichungsformen (Oral Solid Dosages OSD) sein. Die Anlage weist einen an einem Produkteinlass angeordneten Einlassmassesensors auf, der das Gewicht bzw. die Masse des der Anlage zugeführten Produkts bestimmt. Auf Grundlage dieser Massebestimmung wird das der Anlage zugeführte Produkt beispielsweise in einer Auswerte- und Steuereinrichtung der Anlage in aufeinanderfolgende Masseeinheiten aufgeteilt. Die Anlage weist mindestens einen weiteren Massesensor auf, der dem mindestens einen Einlassmassesensor in Fließrichtung des Produkts nachgeordnet ist, und der die Masse bzw. das Gewicht des durch die Anlage fließenden Produkts bestimmt. Auf dieser Grundlage wird der Fortgang der Masseeinheiten in der Anlage verfolgt. Diese Produktverfolgung erfolgt insbesondere mittels einer Auswerteeinrichtung der Anlage, vorzugsweise auf Grundlage von Auswertealgorithmen. Die Massesensoren können die Masse dabei direkt messen, also eine Wiegeeinrichtung umfassen, oder direkt den Massenstrom messen. Sie können die Masse oder den Massenstrom aber auch indirekt messen.

Die Erfindung schlägt also im Gegensatz zum Stand der Technik keine zeitbasierte, sondern eine massebasierte Produktverfolgung vor. Die Masseeinheiten bzw. Massequanten sind klein genug, um eine für den jeweiligen Anwendungszweck ausreichende Genauigkeit der Produktverfolgung zu gewährleisten. Beispielsweise können die Masseeinheiten eine Größe von weniger als 50 g aufweisen. Insbesondere können Schwankungen der Flussgeschwindigkeit bzw. Produktionsgeschwindigkeit (in) der Anlage eine geringere Frequenz besitzen als die Frequenz der aufeinanderfolgenden Masseeinheiten. Dadurch wird die Produktverfolgung weitgehend unabhängig von Schwankungen der Fluss- bzw. Produktionsgeschwindigkeit und somit entsprechenden Abweichungen von einem bestimmten Betriebspunkt der Anlage. Die Produktverfolgung, beispielsweise die Erfassung der Masseeinheiten am Auslass einer Tablettenpresse oder einer Kapselfüllmaschine bzw. der Anlage, und die darauf basierende Zuordnung zu einem der Anlage zugeführten Produkt bzw. einer Produktcharge ist dadurch zuverlässig und bei Erkennen einer fehlerhaften Produktcharge unter Minimierung des Ausschusses möglich.

Die Masseeinheiten werden in der Auswerteeinheit ähnlich einem Schieberegistermodell durch die Anlage bewegt. Es hat sich herausgestellt, dass der Massebezug bei der erfindungsgemäßen Produktverfolgung bereits implizit Eigenschaften aufweist, die in einem zeitbasierten Modell (Verweilzeitmodell) nur durch Verwendung von Gleichungen höherer Ordnung in entsprechenden Auswertealgorithmen erreicht werden können. Die Anzahl der notwendigen Parameter zur Anpassung an ein bestimmtes Produkt bzw. einen Betriebspunkt der Anlage wird dadurch gegenüber einem konventionellen Verweilzeitmodell erheblich reduziert. Gleichzeitig ist es erfindungsgemäß möglich, Materialeigenschaften, die nicht die Konzentration oder Verweilzeit in einem Anlageteil betreffen, ebenfalls zu beobachten. Dies ergibt sich aus der Grundstruktur der erfindungsgemäßen Produktverfolgung ähnlich einem Schieberegister für endliche Masseeinheiten. So ist es im Extremfall möglich, eine einzige Masseeinheit als defekt oder für einen Musterzug zu markieren und zu einem späteren Zeitpunkt aus der Anlage zu entnehmen.

Nach einer bevorzugten Ausgestaltung kann der Fortgang der Masseeinheiten in der Anlage in Echtzeit verfolgt werden. Auf diese Weise wird ein Abgleich des Produktverfolgungsmodells, insbesondere der entsprechenden Algorithmen, mit weiteren Sensordaten der Anlage ermöglicht. Die Robustheit und die Langzeitstabilität des Produktverfolgungsmodells können dadurch weiter erhöht werden.

Wie bereits erwähnt, kann die Anlage eine Anlage zur Tablettenherstellung sein, in der pulverförmiges Produkt in einer Tablettenpresse zu Tabletten verpresst wird. Die Anlage kann also eine Tablettenpresse, zum Beispiel eine Rundläufertablettenpresse, umfassen.

Nach einer weiteren Ausgestaltung kann der Fortgang der Masseeinheiten in der Anlage anhand von Messdaten eines an einem Tablettenauslass der Tablettenpresse angeordneten weiteren Massesensors verfolgt werden. Dieser weitere Massesensor kann zum Beispiel ein Sensor sein, mit dem die Anzahl der aus der Tablettenpresse ausgegebenen Tabletten gezählt wird. Deren Gewicht kann für einen vorgegebenen Prozess als bekannt angenommen werden. Durch die Tablettenzählung kann also eine indirekt Gewichts- bzw. Massebestimmung erfolgen. Es wäre aber natürlich auch möglich, dass der weitere Massesensor die ausgegebenen Tabletten zählt und wiegt. Bei der vorgenannten Ausgestaltung werden die hergestellten Tabletten direkt einer entsprechenden Masseeinheit am Einlass und damit einer entsprechenden Produktcharge zugeordnet.

Gemäß einer weiteren Ausgestaltung kann der Fortgang der Masseeinheiten in der Anlage anhand von Messdaten eines an einer Fülleinrichtung der Tablettenpresse angeordneten weiteren Massesensors verfolgt werden. Dieser Massesensor kann wiederum direkt oder indirekt die Masse oder den Massenstrom messen. Die Fülleinrichtung einer Tablettenpresse besitzt in der Regel ein Füllrohr, über das das pulverförmige Produkt einer Füllkammer zugeführt wird, aus der es in Kavitäten einer Matrizenscheibe der Presse gefüllt wird, wo es dann durch Ober- und Unterstempel der Tablettenpresse verpresst wird. Dem Füllrohr vorgeordnet sein kann ein Füllreservoir. Der weitere Massesensor kann zum Beispiel an dem Füllrohr, dem Füllreservoir oder der Füllkammer angeordnet sein.

Die Anlage kann auch eine Anlage zur Kapselherstellung sein, in der pulverförmiges Produkt in einer Kapselfüllmaschine in Kapseln gefüllt wird. Die Anlage kann dann entsprechend eine Kapselfüllmaschine umfassen.

Der Fortgang der Masseeinheiten in der Anlage kann dann zum Beispiel anhand von Messdaten eines an einem Auslass der Kapselfüllmaschine angeordneten weiteren Massesensors verfolgt werden. Dieser weitere Massesensor kann zum Beispiel ein Sensor sein, mit dem die Anzahl der aus der Kapselfüllmaschine ausgegebenen Kapseln gezählt wird. Deren Gewicht kann für einen vorgegebenen Prozess als bekannt angenommen werden. Durch die Kapselzählung kann also wiederum eine indirekt Gewichts- bzw. Massebestimmung erfolgen. Es wäre aber natürlich auch möglich, dass der weitere Massesensor die ausgegebenen Kapseln zählt und wiegt.

Nach einer weiteren Ausgestaltung kann vorgesehen sein, dass die Anlage mehrere Produkteinlässe für unterschiedliche pulverförmige Produkte und eine Mischeinrichtung zum Mischen der unterschiedlichen Produkte vor dem Verarbeiten zu Erzeugnissen, beispielsweise vor dem Verpressen zu Tabletten in einer Tablettenpresse oder vor dem Befüllen von Kapseln in einer Kapselfüllmaschine umfasst, wobei das in die Anlage eingegebene Produkt anhand von Messdaten mehrerer an den Produkteinlässen der Anlage angeordneten Einlassmassesensoren in Masseeinheiten aufgeteilt wird.

Der Fortgang der Masseeinheiten in der Anlage kann dann anhand von Messdaten mindestens eines an der Mischeinrichtung angeordneten weiteren Massesensors verfolgt werden. Unterschiedliche, der Anlage zugeführte Produkte können zum Beispiel ein aktiver pharmazeutischer Wirkstoff (Active Pharmaceutical Ingredient API), ein Trägermaterial (Exzipient) und/oder ein Schmiermittel sein. Die unterschiedlichen Produkte werden der Anlage über getrennte Einlässe zugeführt, von denen sie der Mischeinrichtung zugeführt werden, wo sie zu der zu verpressenden Produktmischung gemischt werden. Die Mischeinrichtung kann zum Beispiel eine Mischschnecke umfassen. Dadurch erfolgt ein gleichzeitiges Fördern des Produkts beim Mischen.

Nach einer weiteren Ausgestaltung kann für die Verfolgung des Fortgangs der Masseeinheiten in der Anlage ein Vermischen unterschiedlicher Masseeinheiten zu neuen Masseeinheiten in der Mischeinrichtung berücksichtigt werden. Für die Berücksichtigung des Vermischens kann ein durch die Mischeinrichtung realisiertes Mischungsverhältnis berücksichtigt werden. Es werden also aus den bei der Mischeinrichtung ankommenden Masseeinheiten neue Masseeinheiten gebildet, die das gemischte Produkt enthalten. Das Mischungsverhältnis der Mischeinrichtung ist dabei abhängig von der Spezifikation der Mischeinrichtung und gegebenenfalls der Zuführrate der einzelnen Produkte. Das Mischungsverhältnis ist für den jeweiligen Prozess also bekannt und kann für die Berücksichtigung des Vermischens herangezogen werden. Wenn die Mischeinrichtung zum Beispiel zwei Produkte gleichförmig im Verhältnis 50 % zu 50 % mischt, enthalten die neuen Masseeinheiten jeweils das erste und das zweite Produkt zur Hälfte. Bei anderen, durch die Mischeinrichtung realisierten Mischungsverhältnissen erfolgt entsprechend eine andere Aufteilung auf die neuen Masseeinheiten. In der Mischeinrichtung vermischen sich die Masseeinheiten untereinander, abhängig von dem bekannten Mischungsverhältnis. Dies kann zum Beispiel berücksichtigt werden, indem über aufeinanderfolgende Masseeinheiten ein gleitender Mittelwert gebildet wird und aus dem jeweils gebildeten Mittelwert neue Masseeinheiten gebildet werden. Auf diese Weise kann berechnet werden, in welchem Maße bestimmte Masseeinheiten Bestandteile aus unterschiedlichen Produktchargen von den unterschiedlichen Produkteinlässen aufweisen.

Ein Vermischen bzw. eine Dispersion kann neben der Mischeinrichtung auch in weiteren Komponenten der Anlage auftreten, in denen ein Rührwerk Produktchargen vermischt oder zum Beispiel in einer Fülleinrichtung einer Tablettenpresse. Für die Verfolgung des Fortgangs der Masseeinheiten in der Anlage kann entsprechend ein Vermischen unterschiedlicher Masseeinheiten zu neuen Masseeinheiten auch in anderen Komponenten der Anlage, zum Beispiel in Dosierzuführeinrichtungen oder einer Tablettenpresse oder einer Kapselfüllmaschine berücksichtigt werden. Für die Berücksichtigung des Vermischens kann wiederum ein durch die jeweilige Komponente realisiertes Mischungsverhältnis berücksichtigt werden. Wiederum werden aus den bei der jeweiligen Komponente ankommenden Masseeinheiten neue Masseeinheiten gebildet, die das gemischte Produkt enthalten. Das Mischungsverhältnis der Komponente ist dabei in der Regel für den jeweiligen Prozess bekannt und kann für die Berücksichtigung des Vermischens herangezogen werden, wie dies zur Mischeinrichtung erläutert wurde.

Nach einer weiteren Ausgestaltung kann für die Verfolgung des Fortgangs der Masseeinheiten in der Anlage eine Rückvermischung unterschiedlicher Masseeinheiten berücksichtigt werden. Eine solche Rückvermischung, bei der das Produkt zwischen unterschiedlichen Masseeinheiten wechselt, beispielsweise aus einer Masseeinheit in eine vorangehende Masseeinheit eintritt, kann gewollt oder nicht gewollt sein. Sie ist jedenfalls in der Regel spezifisch für die jeweilige Anlage. Sie kann somit vorab empirisch ermittelt werden und dann in den Algorithmen für die Produktverfolgung berücksichtigt werden. Für die empirische Ermittlung der Rückvermischung können zum Beispiel Versuche anhand von Spektrometrie erfolgen. Auch denkbar ist der Einsatz eines sogenannten Tracermaterials, das eindeutig rückverfolgt werden kann, beispielsweise aufgrund einer bestimmten Farbgebung.

Gemäß einer weiteren Ausgestaltung können für die Verfolgung des Fortgangs der Masseeinheiten auch Toträume für Produkt in der Anlage berücksichtigt werden. In derartigen Toträumen bzw. Totzonen kann sich Produkt sammeln, das dann verspätet aus der Anlage austritt. Solche Toträume treten beispielsweise in Schneckenförderern zum Weiterfördern des Produkts in der Anlage auf. Sie können wiederum empirisch vermittelt werden, da sie ebenfalls in der Regel spezifisch für die jeweilige Anlage sind, und dann in den für die Produktverfolgung verwendeten Algorithmen berücksichtigt werden.

Nach einer weiteren Ausgestaltung kann für die Verfolgung des Fortgangs der Masseeinheiten in der Anlage ein Produktverlust in der Anlage, beispielsweise ein Produktverlust durch eine Absaugeinrichtung der Anlage, berücksichtigt werden. Insbesondere bei langen Laufzeiten der Anlage ist ein solcher Produktverlust signifikant und muss durch mehr Materialnachschub ausgeglichen werden. Bei zeitbasierten Verweilzeitmodellen müssten die Parameter des Modells hierzu aufwendig geändert werden. Dies ist erfindungsgemäß nicht erforderlich. Der Produktverlust kann ermittelt werden, indem für eine vorgegebene Produktmenge der Massenstrom in die Anlage und aus der Anlage, beispielsweise die Anzahl sowie das Gewicht von pro Zeiteinheit aus der Anlage austretenden Erzeugnissen, zum Beispiel Tabletten oder Kapselfüllmaschinen, verglichen wird. Produktverlust kann bei einer üblichen Absaugung von zum Beispiel Tablettenpressen zum Aufrechterhalten eines Unterdrucks in der Tablettenpresse auftreten, wenn neben Luft auch etwas Produkt abgesaugt wird. Wiederum ist dieser Produktverlust in der Regel spezifisch für die jeweilige Anlage und kann entsprechend empirisch ermittelt werden, zum Beispiel in der oben erläuterten Weise.

Die Massen der Masseeinheiten können nach einer bevorzugten Ausgestaltung gleich sein. Wenn alle Masseeinheiten die gleiche Masse besitzen, vereinfacht dies die Rückverfolgung, insbesondere die zur Produktverfolgung verwendeten Algorithmen.

Wie eingangs erläutert, weisen die Masseeinheiten eine ausreichend geringe Masse auf für eine zuverlässige und präzise Produktverfolgung. Nach einer diesbezüglichen Ausgestaltung kann die Masse der Masseeinheiten in einem Bereich von 1 g bis 20 g, vorzugsweise in einem Bereich von 1 g bis 10 g liegen. Insbesondere können dabei alle Masseeinheiten die gleiche Masse besitzen, wobei diese für alle Masseeinheiten gleiche Masse dann eine Masse in den genannten Bereichen ist. Die Masseeinheiten sind somit ausreichend klein für eine präzise Produktverfolgung und gegebenenfalls eine gezielte Aussortierung von Erzeugnissen einer fehlerhaften Masseeinheit unter Vermeidung größerer Mengen an Ausschuss.

Nach einer weiteren Ausgestaltung kann anhand von Messdaten mehrerer Massesensoren der Anlage eine Plausibilitätsbewertung der Verfolgung des Fortgangs der Masseeinheiten in der Anlage erfolgen. Bei Auswertung mehrerer Messeinrichtungen zur Ermittlung der Masse der Masseeinheiten kann eine fehlerhafte Massebestimmung erkannt werden, beispielsweise wenn bei der Verfolgung der Masseeinheiten ein Drift festgestellt wird.

Der mindestens eine Produkteinlass der Anlage kann mindestens eine Dosierzuführeinrichtung umfassen, insbesondere eine Loss-In-Weight-Dosierzuführeinrichtung. Derartige Dosierzuführeinrichtungen dienen zum dosierten Zuführen von Produkt. Sogenannte Loss-In-Weight-Dosierzuführeinrichtungen (LIW Feeder) weisen eine Wiegeeinrichtung auf und dosieren das zuzuführende Produkt über eine Gewichtsmessung des in der Dosierzuführeinrichtung befindlichen Produkts. Diese Wiegeeinrichtung kann in besonders praxisgemäßer Weise als Einlassmassesensor genutzt werden.

Wie bereits erläutert, eignet sich die Erfindung insbesondere für Anlagen zur kontinuierlichen Verarbeitung des pulverförmigen Produkts zu Erzeugnissen, beispielsweise zur kontinuierlichen Tablettenherstellung oder Kapselfüllung. Wie an sich bekannt, wird bei Anlagen zur kontinuierlichen Herstellung das Produkt im Gegensatz zu einem Batch-Prozess kontinuierlich in die Anlage zugeführt und in dieser kontinuierlich zu Erzeugnissen verarbeitet, gegebenenfalls nach vorherigem kontinuierlichem Mischen des Produkts. Solche Anlagen können theoretisch endlos laufen, wobei lediglich für einen ausreichenden Produktnachschub an den Produkteinlässen gesorgt werden muss. Sie haben gegenüber Batch-Anlagen diverse Vorteile, wie dem Fachmann an sich bekannt ist. Gerade bei solchen Anlagen zur kontinuierlichen Herstellung ist eine präzise und hochaufgelöste Produktverfolgung, wie sie erfindungsgemäß gewährleistet ist, von großer Bedeutung.

Weiterhin kann die Anlage eine sogenannte Containment-Anlage sein, die also aufgrund besonderer Abdichtungsmaßnahmen einen Austritt von Produktstaub aus der Anlage weitgehend unterbindet. Der Containment-Level einer Anlage wird zum Beispiel nach dem sogenannten SMEPAC Test ermittelt (Standardized Measurement of Equipment Particulate Airborne Concentration). Die bei dem erfindungsgemäßen Verfahren verwendete Anlage kann zum Beispiel einen Containment-Level nach dem SMEPAC Test von 10 - 100 $\mu$g/m$^3$ oder kleiner als 10 $\mu$g/m$^3$ besitzen.

Ein Ausführungsbeispiel der Erfindung wird nachfolgend anhand von Figuren näher erläutert.

Es zeigen schematisch:

Figur 1     eine bei dem erfindungsgemäßen Verfahren verwendete Anlage zur Herstellung von Tabletten mit einer Tablettenpresse in abgewickelter Darstellung des Rotors, und

Figur 2     ein Diagramm zur Veranschaulichung des erfindungsgemäßen Verfahrens.

Sofern nichts anderes angegeben ist, bezeichnen in den Figuren gleiche Bezugszeichen gleiche Gegenstände.

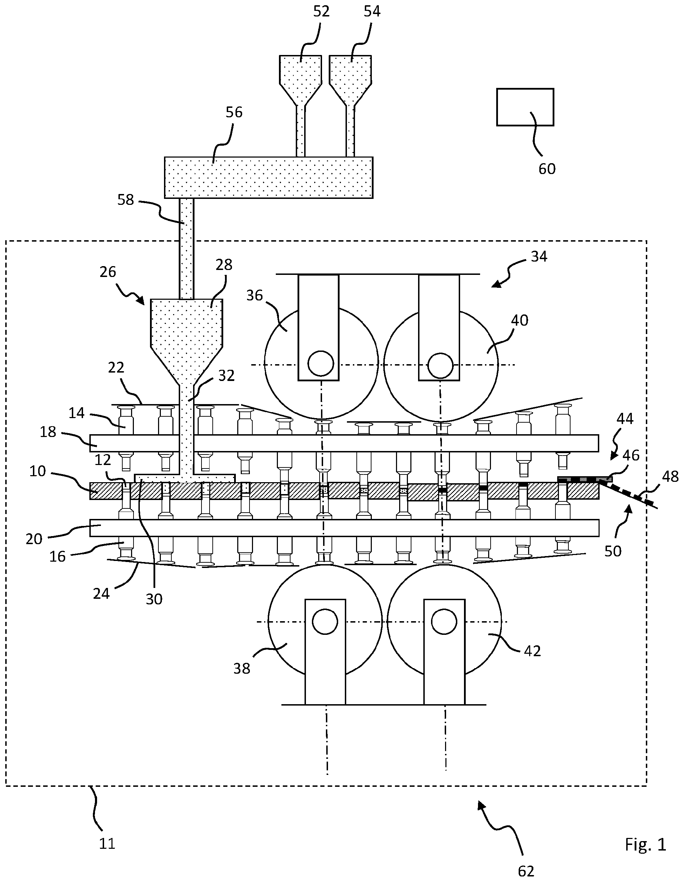

Die in Figur 1 gezeigte Anlage ist eine Anlage zur Tablettenherstellung, in der pulverförmiges Produkt zu Tabletten verpresst wird. Die Anlage umfasst entsprechend eine in einem Gehäuse 11 angeordnete Rundläuferpresse, insbesondere Rundläufertablettenpresse, mit einem durch einen Drehantrieb drehend angetriebenen Rotor mit einer Matrizenscheibe 10, die eine Mehrzahl von Aufnahmen 12 aufweist. Die Aufnahmen 12 können beispielsweise durch Bohrungen der Matrizenscheibe 10 gebildet sein. Weiter umfasst der Rotor eine Mehrzahl von Oberstempeln 14 und Unterstempeln 16, die mit der Matrizenscheibe 10 synchron umlaufen. Die Oberstempel 14 sind in einer oberen Stempelführung 18 axial geführt und die Unterstempel 16 sind in einer unteren Stempelführung 20 axial geführt. Die axiale Bewegung der Oberstempel 14 und Unterstempel 16 im Zuge der Drehung des Rotors wird durch obere Steuerkurvenelemente 22 und untere Steuerkurvenelemente 24 gesteuert. Die Rundläuferpresse umfasst weiterhin eine Fülleinrichtung 26, die ein Füllreservoir 28 und eine Füllkammer 30 aufweist, die über ein Füllrohr 32 verbunden sind. Auf diese Weise gelangt in dem vorliegenden Beispiel pulverförmiges Füllmaterial aus dem Füllreservoir 28 über das Füllrohr 32 schwerkraftbedingt in die Füllkammer 30 und aus dieser über eine an der Unterseite der Füllkammer 30 vorgesehene Befüllöffnung wiederum schwerkraftbedingt in die Aufnahmen 12 der Matrizenscheibe 10.

Die Rundläuferpresse umfasst außerdem eine Druckeinrichtung 34. Die Druckeinrichtung 34 umfasst eine Vordruckeinrichtung mit einer oberen Vordruckrolle 36 und einer unteren Vordruckrolle 38, sowie eine Hauptdruckeinrichtung mit einer oberen Hauptdruckrolle 40 und einer unteren Hauptdruckrolle 42. Darüber hinaus umfasst die Rundläuferpresse eine Auswurfeinrichtung 44 und eine Abstreifeinrichtung 46 mit einem Abstreifelement, das die in der Rundläuferpresse hergestellten Tabletten 48 einer Ablaufeinrichtung 50 zum Abführen aus der Rundläuferpresse zuführt. Die Abstreifeinrichtung 46 kann zum Beispiel ein vorzugsweise sichelförmiges Abstreifelement umfassen, das im Bereich der Auswurfeinrichtung 44 durch die Unterstempel 16 auf die Oberseite der Matrizenscheibe 10 geförderte Tabletten 48 von der Matrizenscheibe 10 abstreift und der Ablaufeinrichtung 50 zuführt.

Das Gehäuse 11 kann gegenüber der Umgebung des Gehäuses 11 unter einem Über- oder Unterdruck stehen. Außerdem kann das Gehäuse 11 dicht gegenüber der Umgebung abgeschlossen sein. Die Rundläuferpresse kann eine sogenannte Containment-Presse sein.

Es wird ausdrücklich darauf hingewiesen, dass die in Figur 1 gezeigte Rundläuferpresse mit ihren erläuterten Eigenschaften lediglich beispielhaft ist. Für die Erfindung ist grundsätzlich auch jede andere Tablettenpresse geeignet. Auch ist für die Erfindung grundsätzlich jede andere Art von Produktionsmaschine geeignet, in der pulverförmiges Produkt zu Erzeugnissen verarbeitet wird, wie beispielsweise eine Kapselfüllmaschine, in der pulverförmiges Produkt in Kapseln gefüllt wird.

Die Anlage umfasst darüber hinaus in dem dargestellten Beispiel zwei Produkteinlässe 52, 54 für zwei unterschiedliche, der Tablettenpresse zum Verpressen zu Tabletten 48 zuzuführende Produkte, beispielsweise einen pharmazeutischen Wirkstoff einerseits und einen Exzipienten andererseits. Die Produkteinlässe 52, 54 können zum Beispiel Dosierzuführeinrichtungen, insbesondere Loss-In-Weight-Dosierzuführeinrichtungen, umfassen. Von den Produkteinlässen 52, 54 gelangen die zugeführten Produkte zu einer Mischeinrichtung 56 der Anlage, in der die Produkte zu der zu verpressenden Produktmischung gemischt werden. Die Mischeinrichtung kann zum Beispiel eine Mischschnecke umfassen. Über eine Zuführleitung 58 wird das aus der Mischeinrichtung 56 austretende gemischte Produkt dem Füllreservoir 28 der Fülleinrichtung 26 zugeführt. Die Anlage umfasst außerdem eine Auswerte- und Steuereinrichtung 60 zum Steuern des Betriebs der Anlage und zur Durchführung der erfindungsgemäßen Produktverfolgung auf Grundlage von in der Auswerte- und Steuereinrichtung 60 hinterlegten Auswertealgorithmen. Die Produkteinlässe 52, 54, die Mischeinrichtung 56, die Fülleinrichtung 26, die Tablettenpresse, insbesondere ein Tablettenauslass der Tablettenpresse, können jeweils mindestens einen Massesensor umfassen, mit dem die Masse bzw. das Gewicht von durch die Anlage geführtem Produkt bzw. aus der Anlage ausgegebenen Tabletten direkt oder indirekt bestimmt werden kann. Die Auswerte- und Steuereinrichtung 60 ist mit den Massesensoren und gegebenenfalls mit weiteren Sensoren der Anlage verbunden. Insbesondere empfängt sie Messdaten der Sensoren und legt sie der Steuerung und Auswertung zu Grunde. Dazu kann die Auswerte- und Steuereinrichtung 60 mit sämtlichen Komponenten der Anlage über entsprechende Verbindungsleitungen verbunden sein.

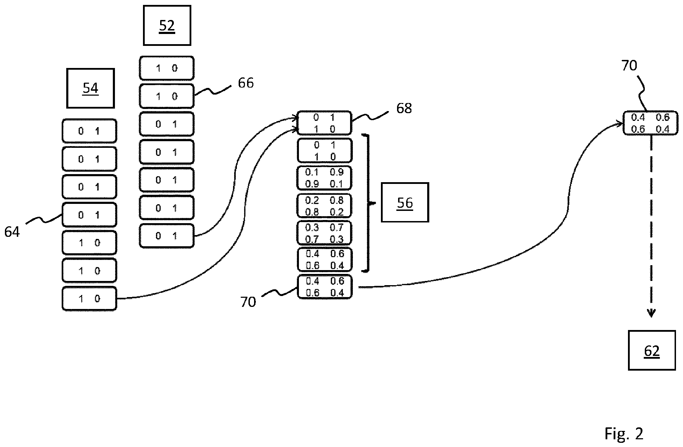

Anhand der Figur 2 soll das erfindungsgemäße Verfahren zur Produktverfolgung in der in Figur 1 gezeigten Anlage näher erläutert werden. Dabei sind die Produkteinlässe 52, 54, die Mischeinrichtung 56 und bei dem Bezugszeichen 62 die Tablettenpresse äußerst schematisch dargestellt. Anhand von Messdaten von an den Produkteinlässen 52, 54 angeordneten Einlassmassesensoren wird das über die Produkteinlässe 52, 54 in die Anlage eingegebene Produkt jeweils in gleichgroße Masseeinheiten 64, 66 aufgeteilt. Dabei sind Masseeinheiten 64, 66 einer ersten Produktcharge mit den Zahlen 0 1 gekennzeichnet und Masseeinheiten 64, 66 einer zweiten Produktcharge mit den Zahlen 1 0. Am Beginn der durch die Mischeinrichtung 56 gebildeten Dispersionszone werden aus den Masseeinheiten 64, 66 von den beiden Produkteinlässen 52, 54 neue Masseeinheiten 68 gebildet, in denen entsprechend dem bekannten Zuführverhältnis in die Mischeinrichtung 56 Produktanteile aus jeweils zwei Masseeinheiten 64, 66 von den beiden Produkteinlässen 52, 54 enthalten sind. In Figur 2 ist das Zuführverhältnis beispielhaft für beide Produkteinlässe 52, 54 als gleich angenommen. Beispielsweise für die in Figur 2 an der unter den Produkteinlässen 52, 54 befindlichen Masseeinheitenketten ganz unten angeordneten Masseeinheiten 64, 66 bedeutet dies, dass aus diesen zwei Masseeinheiten 64, 66 zwei neue Masseeinheiten 68 gebildet werden, die jeweils zur Hälfte aus den Ausgangsmasseeinheiten 64, 66 gebildet sind. Die so gebildeten Masseeinheiten 68 sind entsprechend ihrer Zusammensetzung mit den Zahlen $\frac{0\ 1}{1\ 0}$ gekennzeichnet. Ihre Masse besteht also jeweils zur Hälfte aus Produkt aus der ersten Produktcharge 0 1 vom ersten Produkteinlass 52 und aus Produkt aus der zweiten Produktcharge 1 0 vom zweiten Produkteinlass 54.

Entsprechend dem ebenfalls bekannten, beispielsweise empirisch ermittelten Mischungsverhältnis der Mischeinrichtung 56 wird das Vermischen der Bestandteile der Masseeinheiten in der durch die Mischeinrichtung 56 gebildeten Dispersionszone berücksichtigt, bis zum Erreichen der in Figur 2 bei dem Bezugszeichen 70 zu erkennenden Masseeinheiten. Dabei erfolgt in der Dispersionszone eine Vermischung auch aufeinanderfolgender Masseeinheiten 68 miteinander. Dadurch kann es auch zu einer Vermischung von Produkt aus den unterschiedlichen Ausgangsproduktchargen an den Produkteinlässen 52, 54 kommen. Mathematisch kann die Vermischung in der Dispersionszone zum Beispiel durch Bilden eines gleitenden Mittelwerts aus aufeinanderfolgenden Masseeinheiten 68 als Schieberegister abgebildet werden. Dadurch verändern sich die durch die Zahlenpaare angegebenen Verhältnisse der Bestandteile der Masseeinheiten 68. Am Ende der Dispersionszone sind die gebildeten Masseeinheiten 70 in dem gezeigten Beispiel mit den Zahlen $\frac{0.4\ 0.6}{0.6\ 0.4}$ gekennzeichnet. Dies bedeutet, dass die Masseeinheiten 70 im dargestellten Beispiel 40% Masse aus der zweiten Produktcharge (1 0) des ersten Produkteinlasses 52 und 60% Masse aus der ersten Produktcharge (0 1) des ersten Produkteinlasses 52 aufweisen. Weiterhin bedeutet dies, dass die Masseeinheiten 70 60% Masse aus der zweiten Produktcharge (1 0) des zweiten Produkteinlasses 54 und 40% Masse aus der ersten Produktcharge (0 1) des zweiten Produkteinlasses 52 aufweisen. Es versteht sich, dass diese Verhältnisse sich verändern abhängig davon, zu welchem Anteil sich Produkt aus unterschiedlichen Produktchargen in der Mischeinrichtung 56 befindet.

Die Masseeinheiten 70 werden in ihrem Fortgang durch die Anlage, insbesondere zur Tablettenpresse 62 weiterverfolgt, insbesondere anhand von Messdaten eines zum Beispiel in der Fülleinrichtung 26 der Tablettenpresse angeordneten Massesensors. Über einen weiteren, beispielsweise am Auslass der Tablettenpresse angeordneten Massesensor, der die ausgeworfenen Tabletten zählt und gegebenenfalls zusätzlich ihr Gewicht misst, können die ausgeworfenen Tabletten 48 bestimmten, über die Produkteinlässe 52, 54 zuvor in die Anlage eingegebenen Masseeinheiten 64, 66 und damit der entsprechenden Produktcharge dieser Masseeinheiten 64, 66 zugeordnet werden. Wird beispielsweise eine oder mehrere Masseeinheiten im Zuge ihres Fortgangs durch die Anlage von Sensoren der Anlage als fehlerhaft erkannt, ist es auf diese Weise möglich, die aus diesen Masseeinheiten hergestellten Tabletten zuverlässig zu identifizieren, so dass diese aus den hergestellten Tabletten aussortiert werden können.

Bei der Verfolgung des Fortgangs der Masseinheiten in der Anlage kann auch eine für die Anlage in der Regel spezifische Rückvermischung von Produkt zwischen verschiedenen Masseeinheiten berücksichtigt werden. Das Gleiche gilt hinsichtlich für die Anlage in der Regel spezifische Toträume für Produkt oder ein wiederum in der Regel für die Anlage spezifischer Produktverlust in der Anlage.

Es versteht sich, dass die oben erläuterte Aufteilung des Produkts in die Masseeinheiten und die entsprechende Verfolgung der Masseeinheiten in der Anlage sowie die Zuordnung der hergestellten Tabletten zu bestimmten Masseeinheiten durch die Auswerte- und Steuereinrichtung 60 auf Grundlage von entsprechenden, in Form von Algorithmen hinterlegten Modellen erfolgt.

Die Masseeinheiten 64, 66, 68, 70 können sämtlich die gleiche Masse aufweisen. Die Masse der Masseeinheiten kann vorzugsweise weniger als 20 g, weiter vorzugsweise weniger als 10 g, beispielsweise etwa 1 g betragen. Die Anlage ist insbesondere eine Anlage zur kontinuierlichen Tablettenherstellung. Es kann sich um eine Containment-Anlage handeln.

Bezugszeichenliste

| 10 | Matrizenscheibe |
| 11 | Gehäuse |
| 12 | Aufnahmen |
| 14 | Oberstempel |
| 16 | Unterstempel |
| 18 | Obere Stempelführung |
| 20 | Untere Stempelführung |
| 22 | Obere Steuerkurvenelemente |
| 24 | Untere Steuerkurvenelemente |
| 26 | Fülleinrichtung |
| 28 | Fühlreservoir |
| 30 | Füllkammer |
| 32 | Füllrohr |
| 34 | Druckeinrichtung |
| 36 | Obere Vordruckrolle |
| 38 | Untere Vordruckrolle |
| 40 | Obere Hauptdruckrolle |
| 42 | Untere Hauptdruckrolle |
| 44 | Auswurfeinrichtung |
| 46 | Abstreifeinrichtung |
| 48 | Tabletten |
| 50 | Ablaufeinrichtung |
| 52 | Produkteinlass |
| 54 | Produkteinlass |
| 56 | Mischeinrichtung |

58     Zuführleitung
60     Auswerte- und Steuereinrichtung
62     Tablettenpresse
64     Masseeinheiten
66     Masseeinheiten
68     Masseeinheiten
70     Masseeinheiten

**Patentansprüche**

1. Verfahren zur Produktverfolgung in einer Anlage zur kontinuierlichen Verarbeitung pulverförmigen Produkts zu Erzeugnissen, wobei die Anlage eine Anlage zur Tablettenherstellung ist, in der pulverförmiges Produkt in einer Tablettenpresse zu Tabletten verpresst wird und/oder dass die Anlage eine Anlage zur Kapselherstellung ist, in der pulverförmiges Produkt in einer Kapselfüllmaschine in Kapseln gefüllt wird, **dadurch gekennzeichnet, dass** das in die Anlage eingegebene Produkt anhand von Messdaten mindestens eines an einem Produkteinlass (52, 54) der Anlage angeordneten Einlassmassesensors in Masseeinheiten (64, 66, 68, 70) aufgeteilt wird, und dass der Fortgang der Masseeinheiten (64, 66, 68, 70) in der Anlage anhand von Messdaten mindestens eines weiteren Massesensors der Anlage verfolgt wird.

2. Verfahren nach Anspruch 1, **dadurch gekennzeichnet, dass** der Fortgang der Masseeinheiten (64, 66, 68, 70) in der Anlage in Echtzeit verfolgt wird

3. Verfahren nach einem der vorhergehenden Ansprüche, **dadurch gekennzeichnet, dass** der Fortgang der Masseeinheiten (64, 66, 68, 70) in der Anlage anhand von Messdaten eines an einem Tablettenauslass der Tablettenpresse (62) angeordneten weiteren Massesensors verfolgt wird und/oder dass der Fortgang der Masseeinheiten (64, 66, 68, 70) in der Anlage anhand von Messdaten eines an einer Fülleinrichtung (26) der Tablettenpresse (62) angeordneten weiteren Massesensors verfolgt wird.

4. Verfahren nach einem der vorhergehenden Ansprüche, **dadurch gekennzeichnet, dass** die Anlage mehrere Produkteinlässe (52, 54) für unterschiedliche pulverförmige Produkte und eine Mischeinrichtung (56) zum Mischen der unterschiedlichen Produkte vor dem Verarbeiten zu Erzeugnissen umfasst, wobei das in die Anlage eingegebene Produkt anhand von Messdaten mehrerer an den Produkteinlässen der Anlage angeordneten Einlassmassesensoren in Masseeinheiten (64, 66, 68, 70) aufgeteilt wird.

5. Verfahren nach Anspruch 4, **dadurch gekennzeichnet, dass** der Fortgang der Masseeinheiten (64, 66, 68, 70) in der Anlage anhand von Messdaten mindestens eines an der Mischeinrichtung (56) angeordneten weiteren Massesensors verfolgt wird.

6. Verfahren nach einem der Ansprüche 4 oder 5, **dadurch gekennzeichnet, dass** für die Verfolgung des Fortgangs der Masseeinheiten (64, 66, 68, 70) in der Anlage ein Vermischen unterschiedlicher Masseeinheiten (64, 66, 68, 70) zu neuen Masseeinheiten (64, 66, 68, 70) in der Mischeinrichtung (56) berücksichtigt wird.

7. Verfahren nach Anspruch 6, **dadurch gekennzeichnet, dass** für die Berücksichtigung des Vermischens unterschiedlicher Masseeinheiten (64, 66, 68, 70) zu neuen Masseeinheiten (64, 66, 68, 70) in der Mischeinrichtung (56) ein durch die Mischeinrichtung (56) realisiertes Mischungsverhältnis berücksichtigt wird.

8. Verfahren nach einem der Ansprüche 6 oder 7, **dadurch gekennzeichnet, dass** zur Berücksichtigung des Vermischens unterschiedlicher Masseeinheiten (64, 66, 68, 70) zu neuen Masseeinheiten (64, 66, 68, 70) in der Mischeinrichtung (56) ein gleitender Mittelwert aus aufeinanderfolgenden Masseeinheiten gebildet wird.

9. Verfahren nach einem der vorhergehenden Ansprüche, **dadurch gekennzeichnet, dass** für die Verfolgung des Fortgangs der Masseeinheiten (64, 66, 68, 70) in der Anlage eine Rückvermischung unterschiedlicher Masseeinheiten (64, 66, 68, 70) berücksichtigt wird, vorzugsweise dass eine durch die Anlage bedingte Rückvermischung empirisch ermittelt wird.

10. Verfahren nach einem der vorhergehenden Ansprüche, **dadurch gekennzeichnet, dass** für die Verfolgung des Fortgangs der Masseeinheiten (64, 66, 68, 70) in der Anlage Toträume für Produkt in der Anlage berücksichtigt werden und/oder dass für die Verfolgung des Fortgangs der Masseeinheiten (64, 66, 68, 70) in der Anlage ein Produktverlust in der Anlage, insbesondere ein Produktverlust durch eine Absaugeinrichtung der Anlage, berücksichtigt wird.

11. Verfahren nach Anspruch 10, **dadurch gekennzeichnet, dass** der Produktverlust ermittelt wird, indem für eine vorgegebene Produktmenge der Massenstrom in die Anlage und die Anzahl sowie das Gewicht von aus der Anlage austretenden Erzeugnissen verglichen wird.

12. Verfahren nach einem der vorhergehenden Ansprüche, **dadurch gekennzeichnet, dass** die Massen der Masseeinheiten (64, 66, 68, 70) gleich sind und/oder dass die Masse der Masseeinheiten (64, 66, 68, 70) in einem Bereich von 1 g bis 20 g, vorzugsweise in einem Bereich von 1 g bis 10 g, liegt.

13. Verfahren nach einem der vorhergehenden Ansprüche, **dadurch gekennzeichnet, dass** anhand von Messdaten mehrerer Massesensoren der Anlage eine Plausibilitätsbewertung der Verfolgung des Fortgangs der Masseeinheiten (64, 66, 68, 70) in der Anlage erfolgt.

14. Verfahren nach einem der vorhergehenden Ansprüche, **dadurch gekennzeichnet, dass** die Anlage eine Containment-Anlage ist.

**Claims**

1. A method for tracking products in a system for continuously processing powdered product into manufactured items, wherein the system is a system for producing tablets, in which powdered product is compressed in a tablet press into tablets and/or the system is a system for producing capsules, in which powdered product is filled in a capsule filling machine into capsules, **characterized in that** the product introduced into the system is divided into mass units (64, 66, 68, 70) on the basis of measurement data of at least one inlet mass sensor arranged at a product inlet (52, 54) of the system, and the progress of the mass units (64, 66, 68, 70) in the system is tracked on the basis of measurement data of at least one further mass sensor of the system.

2. The method according to Claim 1, **characterized in that** the progress of the mass units (64, 66, 68, 70) in the system is tracked in real time.

3. The method according to one of the preceding claims, **characterized in that** the progress of the mass units (64, 66, 68, 70) in the system is tracked on the basis of measurement data of a further mass sensor arranged at a tablet outlet of the tablet press (62) and/or the progress of the mass units (64, 66, 68, 70) in the system is tracked on the basis of measurement data of a further mass sensor arranged on a filling apparatus (26) of the tablet press (62).

4. The method according to one of the preceding claims, **characterized in that** the system comprises multiple product inlets (52, 54) for different powdered products and a mixing apparatus (56) for mixing the different products into manufactured items prior to the processing, wherein the product introduced into the system is divided into mass units (64, 66, 68, 70) on the basis of measurement data of multiple inlet mass sensors arranged at the product inlets of the system.

5. The method according to Claim 4, **characterized in that** the progress of the mass units (64, 66, 68, 70) in the system is tracked on the basis of measurement data of at least one further mass sensor arranged on the mixing apparatus (56).

6. The method according to one of Claims 4 or 5, **characterized in that** a mixing of different mass units (64, 66, 68, 70) into new mass units (64, 66, 68, 70) in the mixing apparatus (56) is taken into account for tracking the progress of the mass units (64, 66, 68, 70) in the system.

7. The method according to Claim 6, **characterized in that** for taking into account the mixing of different mass units (64, 66, 68, 70) into new mass units (64, 66, 68, 70) in the mixing apparatus (56), a mixing ratio achieved by the mixing apparatus (56) is taken into account.

8. The method according to one of Claims 6 or 7, **characterized in that** a moving average over sequential mass units is formed in order to take account of the mixing of different mass units (64, 66, 68, 70) into new mass units (64, 66, 68, 70) in the mixing apparatus (56).

9. The method according to one of the preceding claims, **characterized in that** a backmixing of different mass units (64, 66, 68, 70) is taken into account for tracking the progress of the mass units (64, 66, 68, 70) in the system, preferably that a backmixing caused by the system is determined empirically.

10. The method according to one of the preceding claims, **characterized in that** dead spaces for product in the system are taken into account for tracking the progress of the mass units (64, 66, 68, 70) in the system and/or a product loss in the system, in particular a product loss due to a suction apparatus of the system, is taken into account for tracking the progress of the mass units (64, 66, 68, 70) in the system.

11. The method according to Claim 10, **characterized in that** the product loss is determined by comparing the mass flow into the system for a predefined product quantity and the number as well as the weight of manufactured items exiting from the system.

12. The method according to one of the preceding claims, **characterized in that** the masses of the mass units (64, 66, 68, 70) are the same and/or the mass of the mass units (64, 66, 68, 70) lies in a range from 1 g to 20 g, preferably in a range from 1 g to 10 g.

13. The method according to one of the preceding claims, **characterized in that** the plausibility of the tracking of the progress of the mass units (64, 66, 68, 70) in the system is evaluated on the basis of measurement data of multiple mass sensors of the system.

14. The method according to one of the preceding claims, **characterized in that** the system is a containment system.


**Revendications**

1. Procédé de suivi des produits dans une installation pour la transformation en continu d'un produit pulvérulent en produits fabriqués, l'installation étant une installation pour la fabrication de comprimés, dans laquelle un produit pulvérulent est pressé en comprimés dans une presse à comprimés et/ou l'installation étant une installation pour la fabrication de capsules, dans laquelle un produit pulvérulent est versé dans des capsules dans une machine de remplissage de capsules, **caractérisé en ce que** le produit entré dans l'installation est réparti en unités de masse (64, 66, 68, 70) à l'aide de données de mesure d'au moins un capteur de masse d'entrée disposé à une entrée de produit (52, 54) de l'installation, et **en ce que** la progression des unités de masse (64, 66, 68, 70) dans l'installation est suivie à l'aide de données de mesure d'au moins un capteur de masse supplémentaire de l'installation.

2. Procédé selon la revendication 1, **caractérisé en ce que** la progression des unités de masse (64, 66, 68, 70) dans l'installation est suivie en temps réel.

3. Procédé selon l'une des revendications précédentes, **caractérisé en ce que** la progression des unités de masse (64, 66, 68, 70) dans l'installation est suivie à l'aide de données de mesure d'un capteur de masse supplémentaire disposé à une sortie de comprimés de la presse à comprimés (62) et/ou **en ce que** la progression des unités de masse (64, 66, 68, 70) dans l'installation est suivie à l'aide de données de mesure d'un capteur de masse supplémentaire disposé sur une installation de remplissage (26) de la presse à comprimés (62).

4. Procédé selon l'une des revendications précédentes, **caractérisé en ce que** l'installation comprend plusieurs entrées de produits (52, 54) pour différents produits pulvérulents et un dispositif de mélange (56) pour le mélange des différents produits avant la transformation en produits fabriqués, le produit entré dans l'installation étant réparti en unités de masse (64, 66, 68, 70) à l'aide de données de mesure de plusieurs capteurs de masse d'entrée disposés aux entrées de produits de l'installation.

5. Procédé selon la revendication 4, **caractérisé en ce que** la progression des unités de masse (64, 66, 68, 70) dans l'installation est suivie à l'aide de données de mesure d'au moins un capteur de masse supplémentaire disposé sur le dispositif de mélange (56).

6. Procédé selon l'une des revendications 4 ou 5, **caractérisé en ce qu'**un mélange d'unités de masse différentes (64, 66, 68, 70) en nouvelles unités de masse (64, 66, 68, 70) dans le dispositif de mélange (56) est pris en compte pour le suivi de la progression des unités de masse (64, 66, 68, 70) dans l'installation.

7. Procédé selon la revendication 6, **caractérisé en ce qu'**un rapport de mélange réalisé par le dispositif de mélange (56) est pris en compte pour la prise en compte du mélange d'unités de masse différentes (64, 66, 68, 70) en nouvelles unités de masse (64, 66, 68, 70) dans le dispositif de mélange (56).

8. Procédé selon la revendication 6 ou 7, **caractérisé en ce qu'**une moyenne mobile est formée à partir d'unités de masse consécutives pour la prise en compte du mélange d'unités de masse différentes (64, 66, 68, 70) en nouvelles unités de masse (64, 66, 68, 70) dans le dispositif de mélange (56).

9. Procédé selon l'une des revendications précédentes, **caractérisé en ce qu'**un remélange de différentes unités de masse (64, 66, 68, 70) est pris en compte pour le suivi de la progression des unités de masse (64, 66, 68, 70) dans l'installation, de préférence **en ce qu'**un remélange due à l'installation est déterminé de façon empirique.

10. Procédé selon l'une des revendications précédentes, **caractérisé en ce que** des espaces morts pour produit dans l'installation sont pris en compte pour le suivi de la progression des unités de masse (64, 66, 68, 70) dans l'installation, et/ou **en ce qu'**une perte de produit dans l'installation, en particulier une perte de produit par un dispositif d'aspiration de l'installation est prise en compte pour le suivi de la progression des unités de masse (64, 66, 68, 70) dans l'installation.

11. Procédé selon la revendication 10, **caractérisé en ce que** la perte de produit est déterminée par la comparaison du flux massique dans l'installation et du nombre ainsi que du poids de produits fabriqués sortant de l'installation, pour une quantité de produit spécifiée.

12. Procédé selon l'une des revendications précédentes, **caractérisé en ce que** les masses des unités de masse (64, 66, 68, 70) sont égales et/ou **en ce que** la masse des unités de masse (64, 66, 68, 70) est comprise dans une plage de 1 g à 20 g, de préférence dans une plage de 1 g à 10 g.

13. Procédé selon l'une des revendications précédentes, **caractérisé en ce qu'**une évaluation de plausibilité du suivi de la progression des unités de masse (64, 66, 68, 70) dans l'installation est effectuée à l'aide de données de mesure de plusieurs capteurs de masse de l'installation.

14. Procédé selon l'une des revendications précédentes, **caractérisé en ce que** l'installation est une installation de confinement.

Fig. 1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2427166 B1
- DE 102016218135 A1
- WO 2008149190 A1
- US 5114630 A